# EUROPEAN PATENT APPLICATION

(11) **EP 1 839 688 A1**
(43) Date of publication of application: **03.10.2007**
(21) Application number: 06006367.4
(22) Date of filing: 28.03.2006
(51) Int. Cl.: A61L 31/16, A61L 31/10

(54) **Drug-eluting clinical device**

(71) Applicant: Universität Zürich, 8006 Zürich (CH)
(72) Inventor: Tanner, Felix, CH-8706 Meilen (CH); Camici, Giovanni, CH-8057 Zürich (CH); Lüscher, Thomas, CH-8126 Zumikon (CH)
(74) Representative: Keller, Günter

(57) **Abstract**

The invention relates to implantable clinical devices that contain means for eluting an active ingredient, said active ingredient essentially comprising DMSO, and the use of DMSO for the manufacture of a medicament for the treatment of vascular, cerebrovascular or cardiovascular disorders, acute coronary syndromes, stroke or peripheral artery disease.

## Description

The present invention relates to a clinical device for the treatment of vascular, cerebrovascular and cardiovascular disorders or acute coronary syndromes and the use of DMSO for the manufacture of a medicament for the treatment of vascular, cerebrovascular or cardiovascular disorders or acute coronary syndromes.

Dimethyl sulfoxide (DMSO) is universally employed for preservation of hematopoietic progenitor cells infused into patients after myeloablative therapy (Egorin, 1998) and as a solvent for chemotherapeutic drugs. Moreover, due to its anti-inflammatory properties, DMSO has been successfully employed in treating human pathologies of dermatological (Burgess, 1998; Wong, 1988), neurological (Karaca, 1991, 2002), urinary (McCammon, 1998), pulmonary (lwasaki, 1994), rheumatic (Morassi, 1989) and gastrointestinal (Salim, 1992; 1994) origin. DMSO exhibits protective effects in animal models of mercuric chloride induced kidney injury (Jo, 2004), chemical liver injury (Lind, 1997), middle cerebral artery occlusion (Bardutzky, 2005), and cerebral hypoperfusion related neuronal death (Farkas, 2004). Despite of all this information, the potential impact of DMSO on vascular or cardiovascular disorders has not been assessed.

Tissue Factor (TF) is a key enzyme in the activation of coagulation (Steffel, 2005). Plaque rupture leading to arterial thrombosis is the main cause of acute coronary syndromes (ACS) such as unstable angina and myocardial infarction. TF plays a major role in determining plaque thrombogenicity (Moons, 2002), as it is expressed by different cell types within the atherosclerotic vessel wall and is highly exposed to the blood stream following plaque rupture. Many inflammatory mediators are capable of inducing TF expression in cells such as monocytes, macrophages, endothelial cells, and vascular smooth muscle cells (Moons, 2002). Not surprisingly, experimental evidence has revealed an important contribution of TF to the pathogenesis of restenosis after balloon angioplasty (Kopp, 2004) and thrombosis after drug-eluting stent deployment (Steffel, 2006). As a consequence, recent efforts have focused on the therapeutic implications of tissue factor inhibition (Moons, 2002).

Circulatory diseases are widespread and are related to progressive narrowing of the blood vessel due to atherosclerotic plaques; plaque rupture often leads to ischemic injury, stroke or myocardial infarction. Acute myocardial infarction occurs, because the blood supply to a part of the organ is interrupted; indeed, atherosclerotic lesions, which limit coronary blood flow and eventually rupture, are the major cause of ischemic heart disease.

Percutaneous transluminal angioplasty (PTA) is a medical procedure the purpose of which is to increase blood flow through a blood vessel. PTA of the coronary arteries (PCTA) is featured by a high success rate besides minimal invasiveness compared to coronary bypass surgery. Typically during PTCA a balloon catheter device, which is inserted into the blood vessel, is inflated to dilate the stenotic (abnormally narrowed) blood vessel, which normally results from plaque or plaque rupture leading to thrombosis. During inflation the pressurized balloon exerts a force on the lesion, thereby increasing the inner diameter of the affected vessel. The increased interior vessel diameter facilitates improved blood flow. Soon after the PTCA treatment, however, a significant portion of treated vessels re-narrow.

This re-narrowing, called restenosis, is inibited by introduction of a short flexible cylinder called stent. A stent is either an expandable wire mesh or hollow perforated tube that is inserted into a hollow structure of the body to keep it open.

Although stents are mostly used in treatment of coronary arteries, they can also be used in several other structures, such as peripheral arteries and veins, bile ducts, esophagus, trachea or large bronchie, urethers and urethra.

Restenosis after PTCA treatment remains a major problem in interventional cardiology and is caused by a combination of neointima formation, which is the formation of new tissue, and constrictive vascular remodelling. Although the latter is prevented by a stent, neointima formation can still occur within the stent or at its edges. Hence, to reduce neointima formation in addition to vascular remodeling, drug-eluting stents are known in the art.

EP 1 600 180 A2 discloses a drug delivery device for treating in-stent intimal hyperplasia in human vessel walls comprising a therapeutic dosage of an agent having anti-proliferative and anti-inflammatory properties, which preferably comprises rapamycin.

WO 2004/045578 A2 discloses a drug delivery device comprising a medical device, e.g. a coated stent or stent-graft, in conjunction therewith a therapeutic dosage form of an anti-inflammatory ascomycin derivative, e.g. afixed to the medical device.

However, although at lower rates, neointima formation remains a problem in the art. Furthermore, stent thrombosis is a rare, but much more dangerous complication after coronary stent placement. It usually occurs before endothelialisation has been completed. For bare-metal stents, this process takes a few weeks; the newer, drug-eluting stents inhibit restenosis by inhibiting vascular smooth muscle cell proliferation, but they also tend to delay the endothelialisation process. For this reason, patients who have had drug-eluting stents are prescribed aspirin plus clopidogrel therapy for at least six months. Clopidogrel is a known anti-platelet agent often used in the treatment of coronary artery diseases, peripheral vascular diseases and cerebrovascular diseases. The mechanism of action of clopidogrel is irreversible blockade of the adenosine II phosphate (ADP) receptor P2Y12 on the platelet cell membranes, which is important in platelet aggregation, in particular the crosslinking of platelets by fibrin. The blockade of this receptor inhibits platelet aggregation. Besides adverse effects like severe neutropenia or the occurrence of hemorrhage, in particular occurring when co-administered with aspirin, clopidogrel has the disadvantage of being rather expensive. Therefore, there is still the need for an alternative inhibitor of thrombosis after coronary stent placement; in particular a local inhibitor of thrombosis.

Involvement of tissue factor (TF) in acute coronary syndromes (ACS) is known, as stated above. Recent studies even show increased levels of TF antigen and activity in atherectomy specimens from patients with unstable angina or myocardial infarction as compared to those with stable angina (Annex, 1995). Moreover, in ACS, plasma concentrations of inflammatory cytokines such as TNF-α (tumor necrosis factor α) and interleukins are increased at the site of coronary artery occlusion to such an extent that TF is induced in vascular cells (Maier, 2005).

Therefore, there is not only a need for an inhibitor of neointima formation, but also there is a need for an inhibitor of TF, which can be applied by or in combination with a medical device, e.g. a stent, or by parenteral administration.

The inventors surprisingly found that DMSO is, besides being active in suppression of inflammatory response and inhibition of platelet activity, active in prevention of thrombosis. In particular it was found that DMSO inhibits proliferation and migration of human aortic vascular smooth muscle cells (HASMC), and reduces TF activity *in vitro* and *in vivo* in addition to preventing TF-dependent thrombosis *in vivo.*

Therefore, the present invention relates to a clinical device for being implanted that contains means for eluting an active ingredient, said active ingredient essentially comprising DMSO. Preferably the clinical device according to the present invention comprises an active ingredient, which consists of at least 80% of DMSO, more preferable of at least 95% of DMSO. Percent is given as percent by weight.

All concentrations given in the present application are expressed in terms of weight percent unless designated otherwise

Within this application the terms "eluted" or "eluting" and "released" or "releasing" are used synonymously.

The "clinical device" of the present invention refers to a device which is inserted into, or grafted onto, body tissues to remain for a period of time, such as an extended-released drug delivery device, stent, drug-eluting stent, or vascular graft.

In a preferred embodiment of the present invention a clinical device comprises a surface or surface layer suitable for being implanted.

Preferably the active ingredient of the clinical device of the present invention comprises DMSO, more preferable the active ingredient comprises from about 20 to about 100% DMSO, even more preferred from 60 to 100% DMSO, in particular the active ingredient contains 80 to 90% DMSO. Furthermore, the active ingredient may also comprise other pharmaceutically active compounds like rapamycin, paclitaxel, tacrolimus, statins, and similar compounds, which have beneficial effects for the tissues. DMSO and said other compound(s) add up to 100%.

ln another aspect of the present invention the active ingredient contains at least 20%, preferred at least 50%, more preferred at least 80%, most preferred at least 90%, in particular at least 95% DMSO as active ingredient.

In one embodiment of the present invention the active ingredient of the clinical device comprises DMSO in combination with further anti-proliferative and/or anti-inflammatory agents.

Means for eluting the active ingredient(s) from the clinical device of the present invention are known in the art. Examples of materials that can be applied are layers of drug-eluting materials, such as polymer-matrices. Each polymer suitable for application on a device which can be implanted in an organism can be used, provided that the material is non-toxic and able to release an active ingredient in a pre-defined period of time. Preferred periods of release are from about 2 to 28 days, in particular 14 to 28 days- More preferably the period of release is in a range of 2 to 4 weeks with a maximum of drug-elution after 1 to 2 weeks. In particular, the material to be used as drug-eluting layer has to be able to release an active ingredient, which contains a high amount of DMSO, e.g. from about 50% to about 100% DMSO content, within a predefined period of time.

In one preferred embodiment of the present invention the clinical device is covered with a degradable layer, which is the only or the first layer of the clinical device, said layer comprising one or a variety of polymer matrices. The matrix should be biocompatible, biodegradable, bioerodable, non-toxic, bioabsorbable, and with a defined rate of degradation. Biocompatible matrices that can be used in the invention include, but are not limited to, poly(lactide-co-glycolide), polyesters such as polylactic acid, polyglycolic acid and copolymers thereof, polyanhydride, polycaprolactone, polyhydroxybutyrate, valerate, and other biodegradable polymers and mixtures thereof. In another embodiment the matrix can also comprise naturally occurring polymeric materials such as collagen, fibrin, elastin, and extracellular matrix components.

In another embodiment of the present invention the polymer matrix of the clinical device further comprises pharmaceutically acceptable polymers, as the only or the second layer, for example, non-absorbable polymers, such as ethylene vinyl acetate (EVAC) and methylmethacrylate (MMA). The non-absorbable polymer, for example, can aid in further controlling release of the substance by limiting diffusion, thereby delaying or slowing the rate of release of the active ingredient, in particular when used as second layer.

In one preferred embodiment of the present invention the active ingredient is mainly present in the first, inner polymer matrix layer of the clinical device. This inner polymer matrix layer, containing the active ingredient, which comprises a significant amount of DMSO, can for example be prepared by using DMSO as polymerisation solvent, or the polymer of the inner polymer matrix layer is saturated with DMSO and optionally other ingredients of the active ingredient after polymerisation, before or after application as first polymer matrix layer to the clinical device. Preferably the polymer of this inner layer is a degradable layer as specified above. As second layer, a further, slowly degradable or preferably a non-absorbable polymer layer as defined above can be applied, preferably as diffusion limiting and controlling layer, thereby enabling controlled release of active ingredient, which comprises a significant amount of DMSO, into the surrounding tissue.

In another preferred embodiment of the present invention the active ingredient, comprising a significant amount of DMSO, is contained in pre-formed particles or granules of matrix polymer, which are applied to the clinical device of the present invention, eventually within a further polymer matrix.

The base material of the clinical device of the present invention, which preferably is covered with means for eluting an active ingredient, can be of various types, for example, stainless steel, nitinol, MP35N, gold, tantalum, platinum, platinum-iridium or titanium, which is preferred, or other biocompatible materials and/or alloys such as carbon or carbon fiber, cellulose acetate, cellulose nitrate, silicone, cross-linked polyvinylacetate (PVA), hydrogel, polyurethane, polyamide, polyester, or a mixture of polymers thereof; polyesters such as polylactic acid, polyglycolic acid or copolymers thereof and other biodegradable polymers or suitable degradable materials can also be used.

In one embodiment of the present invention the drug-eluting layer of the clinical device or the clinical device itself is degraded by the organism, which the device is implanted in, thereby releasing the drug contained.

In one preferred embodiment of the present invention the clinical device is a stent, preferably a coated or covered stent, such as those covered with polytetrafluoroethylene (PTFE), expanded polytetrafluoroethylene (ePTFE), and such as those covered stents which are alternatively or additionally covered with a polymer matrix as described above. A stent is either an expandable wire mesh or a hollow perforated tube that is inserted in a hollow structure in the body to keep it open. As base material of the stent all materials as described for the clinical device can be used.

In one embodiment of the present invention the clinical device is a depot stent, which contains the active ingredient in suitable reservoirs or wells, e.g. holes within the stent which are filled with erodable polymer containing the active ingredient.

A bioresorbable drug-delivery stent including a substantially cylindrical expandable stent formed of a bioresorbable material and a plurality of reservoirs or openings formed in the stent containing a beneficial agent matrix comprising a bioresorbable material and drug is for example disclosed in WO 2005/102222, the content of which is incorporated by reference herein.

Because of its lipophilic character DMSO is particularly useful as eluted drug in a stent, as the lipophily renders DMSO suitable for being easily incorporated into suitable non-erodable and/or erodable polymers to be used in a drug-eluting stent of the present invention.

In order to coat a clinical device such as a stent, the stent may be dipped, sprayed with, or loaded with, for example, a liquid solution of the matrix polymer of moderate viscosity. Alternatively, the clinical device such as a stent may be coated with matrix polymer during or after polymerisation, for example by applying the devices into monomer solutions or prepolymerized matrix polymer solutions, preferably in presence of active ingredient, comprising a significant amount of DMSO, to form a layer, covering the device, which is saturated with active ingredient, preferably during polymerisation. A further diffusion limiting and controlling layer as specified above may be applied to the device as second layer. Optionally after application of one layer, the stent is dried before application of the next layer. In one embodiment, a thin, paint-like polymer matrix coating does not exceed an overall thickness of about 100 to about 500 µm.

In one preferred embodiment of the present invention the clinical device is only covered by the drug-eluting layer at those positions, where said device is in direct contact to the organism, i.e. the surrounding tissue.

The "surrounding tissue" according to the present invention is the tissue which is in direct contact to the material of the clinical device of the present invention or the tissue which is in direct contact and the tissue which is directly adjacent to the clinical device of the present invention.

In one preferred embodiment of the present invention the active ingredient, which comprises a significant amount of DMSO, is eluted from the clinical device in an amount effective to significantly suppress expression of tissue factor protein within the tissue surrounding the clinical device. Preferably the expression of tissue factor protein is suppressed by at least 20%, more preferred by at least 30%, in particular by at least 50%, relative to the expression level of tissue factor protein of tissue. which has been exposed to an increased level of inflammatory mediators, comparable to or such as TNF-α or thrombin, in particular compared to or such as concentrations of 5 ng/ml TNF-α or 1 U/ml thrombin. Preferably proliferation and/or migration of HVSMC is suppressed by at least 20%, more preferred at least 30%, in particular by at least 50%, relative to the level when no active ingredient is present.

In one embodiment of the present invention the active ingredient, which comprises at least more than 50% by weight of DMSO, is eluted from the clinical device within a predetermined period of time, preferably during 1-28 days, e.g. for 14 days.

In one embodiment of the present invention the clinical device contains an amount of active ingredient in a range from about 10 ng to about 10 mg, preferably from about 1 µg to about 1000 µg.

In one embodiment of the present invention the active ingredient is incorporated into the drug-eluting layer, in case of two layers in both or one layer, in case of more than two layers in one, two or more layers in a percent loading of between 0.1% to 70% by weight, preferably between 5% and 50% by weight.

In a further preferred embodiment of the present invention the amount eluted from the clinical device into the surrounding tissue is from about 1 ng/mm²h to about 1 µg/mm²h, relative to the contacted surrounding tissue area and time after implantation.

The present invention further relates to the use of DMSO for the manufacture of a medicament for the treatment of vascular, cerebrovascular or cardiovascular disorders, stroke or acute coronary syndromes.

In one embodiment the DMSO used for the manufacture of a medicament for the treatment of vascular, cerebrovascular or cardiovascular disorders, stroke or acute coronary syndromes is used in combination with further anti-proliferative and/or anti-inflammatory agents, e.g. rapamycin, paclitaxel or tacrolimus or in combination with other medicaments applied for treating these conditions.

The medicament for the treatment of vascular, cerebrovascular or cardiovascular disorders, stroke or acute coronary syndromes preferably contains 20-100% DMSO, more preferable 60-100% DMSO, in particular 80-90% DMSO. Said medicament may further comprise appropriate diluents, such as buffer solutions, excipients and stabilizers, being suitable for administration, for example by syringe or infusion. Another possible form of administration is a bolus injection, wherein the DMSO can be premixed with an infusion solution or can be prepared in situ by addition of DMSO into the infusion solution prior to injection. The medicament can be administered parenterally or subcutaneously as desired. When administered systemically, the therapeutic composition should be sterile, pyrogen-free and in a parenterally acceptable solution having due regard for pH, isotonicity, and stability. These conditions are known to those skilled in the art.

In one further embodiment of the present invention the medicament for the treatment of vascular, cerebrovascular or cardiovascular disorders, stroke or acute coronary syndromes can be administered orally, for example in form of a tablet, capsule or any other form suitable for oral administration. Said medicament further comprises appropriate diluents, excipients and coadjuvants suitable for oral administration. Methods for producing these administration forms are known to those skilled in the art.

The present invention further relates to the method of treating vascular, cerebrovascular or cardiovascular disorders, stroke or acute coronary syndromes by administration of drug-eluting stents able to release a drug comprising a significant amount of DMSO and/or administering a medicament comprising a significant amount of DMSO to a patient in need thereof.

In one embodiment of the present invention the method of treating vascular, cerebrovascular or cardiovascular disorders, stroke or acute coronary syndromes comprises administration of a medicament comprising a significant amount of DMSO in a combinational product, preferably by intravenous or oral administration. In particular the medicament comprising a significant amount of DMSO is used in combination with further anti-proliferative and/or anti-inflammatory agents, e.g. rapamycin, paclitaxel or tacrolimus or in combination with other medicaments applied for treating these conditions.

The present invention further relates to a method for reducing restenosis, in-stent restenosis, stent thrombosis or in-stent thrombosis comprising administration of a drug-eluting stent able to release a drug comprising a significant amount of DMSO and/or administering a medicament comprising a significant amount of DMSO to a patient in need thereof.

The present invention further relates to a method of treating acute myocardial infarction, stroke or peripheral arterial disease comprising administering a medicament comprising a significant amount of DMSO to a subject receiving a stent in connection with percutaneous transluminal angioplasty.

Some data obtained by the inventors are summarized in the Figures.

Data presented in the Figures are given as mean±SEM. Statistical analysis was performed by 2-tailed unpaired Student test or ANOVA as appropriate. A probability value of <0.05 was considered significant.
**Figure 1. DMSO inhibits TF expression and activity.**
   Fig. 1A shows a histogram of TNF-α induced TF expression relative to the concentration of DMSO. Values are given as percent of stimulation with TNF-α alone, concentration-dependent manner. Values are given as percent of stimulation with thrombin alone, normalized to GAPDH expression. **P*<0.0001, ***P*<0.001 vs TNF-α alone.
   Fig. 1 B shows a histogram of thrombin-induced TF expression relative to the concentration of DMSO, normalized to GAPDH expression. Values are given as percent of stimulation with thrombin alone. *P<0.001, **P<0.0001 vs thrombin alone.
   Fig. 1C shows a histogram of TF-α induced TF surface activity. Values are given as percent of stimulation with TNF-α alone. **P<0.0001 vs TNF-α alone.
   Fig. 1 D shows Western blots of TNF-α induced TF expression relative to the concentration of DMSO in HAEC, THP-1 and HAVSMC-cells. All blots are normalized to GAPDH expression.
   Values in Figures 1A, 1B and 1C are representative of at least three different experiments.
**Figure 2. Toxicity of DMSO.**
   Fig. 2A shows a histogram of LDH-release of HAEC-cells relative to the concentration of DMSO. Values are given as percent of stimulation with TNF-α alone.
   Fig. 2B shows a histogram of trypan blue cell viability relative to the concentration of DMSO. Values are given as percent of the stimulation with TNF-α alone.
**Figure 3. Effect of DMSO on TF mRNA induction.**
   Fig. 3A shows the histogram of TF/L28 mRNA expression (%) before and after stimulation with TNF-α and after stimulation with TNF-α with DMSO. Values are given as percent of stimulation with TNF-α alone. **P*<0.001, vs TNF-α alone. Values are representative of four different experiments and are normalized to L28 mRNA.
   Fig. 3B shows ΔΔC_{T} values without TNF-α induction and in absence of DMSO (left) and with TNF-α induction and in presence of DMSO (right). **P<0.0001 vs TNF-α alone. Values are representative of four different experiments and are normalized to L28 mRNA.
**Figure 4. Effect of DMSO on MAP kinase activation.**
   Fig. 4A shows Western blots indicating phosphorylation (Pho) of three MAP kinases JNK, p38, and ERK, relative to their total amount (Tot) contained in the cells, with and without DMSO, investigated at several time points after TNF-α induction. Blots are representative of at least three different experiments.
   Fig. 4B shows a histogram showing the activities of the three MAP kinases JNK, p38 and ERK, in presence of DMSO and after induction with TNF-α.
   Fig. 4C shows a histogram indicating TF expression levels without and with TNF-α induction and further addition of SP600125 (SP), a specific inhibitor of JNK, addition of SB203580 (SB), a specific inhibitor of p38, and addition of PD98059 (PD), a specific inhibitor of ERK activation. *P<0.001, vs TNF-α alone. Blots are representative of at least three different experiments.
**Figure 5. Effect of DMSO on TFPI and PAI-1 expression.**
   Fig. 5A shows the TFPI expression level (%) without and with TNF-α induction and with increasing concentrations of DMSO. Values are given as percent of stimulation with TNF-α alone. Blots are representative of at least three different experiments.
   Fig. 5B shows PAI-1 expression levels (%) without and with TNF-α induction and for increasing concentrations of DMSO. Values are given as percent of stimulation with TNF-α alone. **P<0.0001, vs TNF-α alone. Blots are representative of at least three different experiments.
**Figure 6. Effect of DMSO on carotid artery TF activity and thrombotic occlusion of the carotid artery in a mouse model of photochemical injury.**
   Fig. 6A shows a histogram indicating TF activity (absorbance, 405 nm) in the carotid artery in a mouse,model of photochemical injury. ** *P*<0.0001 vs PBS treated.
   Fig. 6B shows blood flow recordings of vehicle treated (PBS), DMSO treated and uninjured mice in a mouse model of photochemical injury. Laser injury is initiated at time = 0 min. This figure shows that DMSO is active not only in vitro but also in vivo.

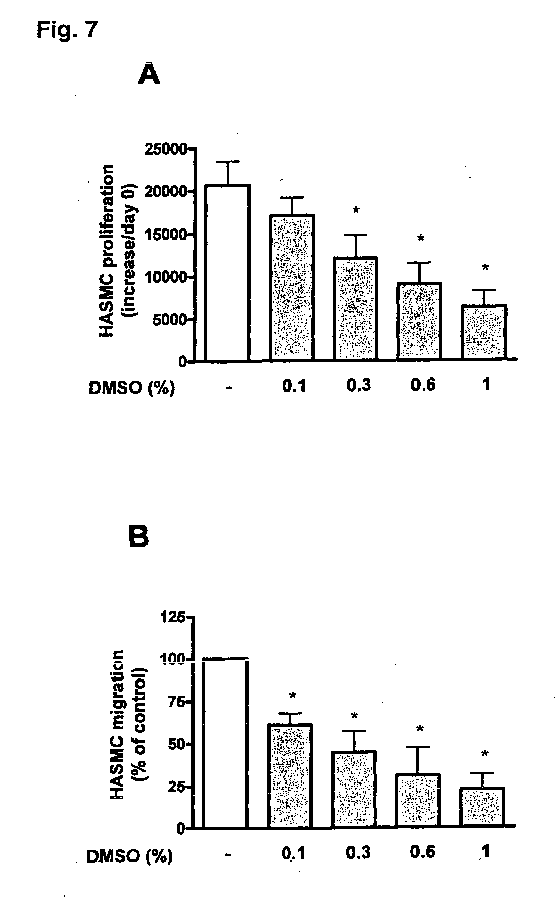

**Figure 7. Effect of DMSO on proliferation and migration of aortic smooth muscle cells.**
   Fig.7A shows a histogram indicating HASMC proliferation for increasing DMSO concentrations.
   Fig. 7B shows a histogram indicating HASMC migration for increasing DMSO concentrations.

The present invention offers a completely new approach to specific inhibition of expression and activity of TF (cp. Example 1). The present inventors found that DMSO is able to inhibit TF induction in human aortic endothelial cells (HAEC), human aortic vascular smooth muscle cells (HAVSMC) and THP-1 cells. This effect was mediated by a reduced activation of p38 and JNK MAP kinase activation (cp. Example 4) and expression of TF mRNA (cp. Example 5). Consistent with this observation, it was shown that DMSO protects from thrombotic occlusion in an *in vivo* model of TF-dependent thrombus formation (cp. Example 6). Further it could be demonstrated that DMSO inhibits proliferation and migration of human aortic smooth muscle cells (HASMC) (cp. Example 7).

These findings denote DMSO as an excellent active ingredient in a variety of applications, preferably in combination with a stent therapy. As DMSO is first active in suppression of inflammatory response, corresponding complications caused by inflammation, in particular shortly after stent implantation can be reduced. Secondly DMSO is also active in prevention of thrombosis, in particular by inhibition of tissue factor and platelet activity. Thrombosis, in particular stent thrombosis, is a rare but mostly very dangerous complication often leading to death of the patient. Thirdly DMSO is a well known solvent which is easy to handle and which is much less expensive than substances with a comparable field of application.

DMSO was used in concentrations as high as 1% by weight (12.6 mmol/L) and no toxic effects by DMSO treatment could be observed, even at the highest concentration (cp. Example 2). In support of these findings, a focused study found no toxic effect by DMSO treatment on endothelial cells at concentrations well above those (Bourne, 1994), and contemporary literature describes routine usage of DMSO to trigger cellular differentiation *in vitro* in concentrations as high as 1.25% (15.7 mM) (Heidari, 2004). Furthermore, DMSO is universally infused intravenously together with hematopoietic progenitor cells in patients after myeloablative therapy and in those patients, DMSO reaches plasma concentrations of 20 mmol/L without causing any adverse effect (Egorin, 1998).

Present inventors showed that thrombin and TNF-α induced TF expression in HAEC with similar potencies, and incubation with DMSO suppressed this induction irrespective of the stimulus. Similarly, DMSO prevented TNF-α induced TF expression in HAVSMC and THP-1 cells.

TF activity is counterbalanced by its endogenous inhibitor TFPI (tissue factor pathway inhibitor); equilibrium between these two factors is known to be essential in determining embryonic survival (Pedersen. 2005). Treatment with DMSO did not affect TPPI expression indicating that the effect of DMSO on TF is not antagonized by a concomitant reduction in TFPI expression. PAI-1 (plasminogen activator inhibitor-1) is a serpin that suppresses fibrinolysis by inhibiting the activity of plasminogen activator. It is known that DMSO is able to prevent interleukin-1 induced PAI-1 expression in rat microvascular endothelial cells (Okada, 1998). By contrast, we did not observe any effect of DMSO on TF expression in TNF-α stimulated HAEC. This difference, maybe related to the different species, the different origin of the cells, or the different stimuli used for induction.

Activation of MAP kinases mediates TF expression in response to several stimuli (Steffel, Akhmedov, 2005; Steffel, Hermann, 2005; Eto, 2002; Mechtcheriakova, 2001). Indeed, p38, ERK, and JNK were transiently activated in HAEC after stimulation with TNF-α, Treatment with DMSO prevented TF expression by specifically decreasing phosphorylation of JNK and p38, but not ERK thus indicating that inhibition of these two MAP kinases reduces TF expression in a potent manner. In line with this observation, it is shown that pharmacological inhibition of just one MAP kinase induces a less pronounced inhibition of TF expression. Consistent with inhibition of MAP kinases, treatment with DMSO led to a strong reduction in TNF-α-induced TF mRNA levels.

It is generally accepted that thrombus formation is triggered by TF (Mackman, 2004; Mann, 1998, Nemerson, 1998). Photochemical injury was selected as a model of thrombosis because it is an established protocol for studying TF *in vivo* (Day, 2005); furthermore, this elegant procedure does not require intra-arterial invasion (Matsuno, 1991; Day, 2004). It was observed by present inventors that DMSO treated mice do not develop thrombotic occlusion following injury, indicating that treatment with DMSO prevents thrombus formation *in vivo.* Inhibition of relatively high basal TF activity in mouse carotid artery *in vivo* occurred within 2 hours of DMSO administration and thus is the most likely explanation for the effect of DMSO treatment on thrombus formation. A recent report is very consistent with this observation by showing that basal arterial TF levels *in vivo* are high and do not undergo a large induction (Day, 2005). In addition, part of the effect displayed by DMSO *in vivo* may also be attributable to its known ability to impair platelet adherence and aggregation (Dujovny, 1983).

Therefore, present inventors showed that DMSO reduces TF activity *in vitro* and *in vivo* in addition to preventing TF-dependent thrombosis *in vivo.* The use of DMSO as a novel strategy for treating ACS is therefore provided. In particular, DMSO can now be used as an attractive alternative for use in conjunction with widely employed immunosuppressant drugs such as rapamycin on stents, especially since rapamycin and paclitaxel has been reported to induce TF expression (Steffel, Latini, 2005, Stahli, submitted).

The following examples further illustrate the invention.

Data presented in the examples are given as mean±SEM. Statistical analysis was performed by 2-tailed unpaired Student *t* test or ANOVA as appropriate. A probability value of <0.05 was considered significant.

### Example 1: Effect of DMSO on TF protein expression and activity

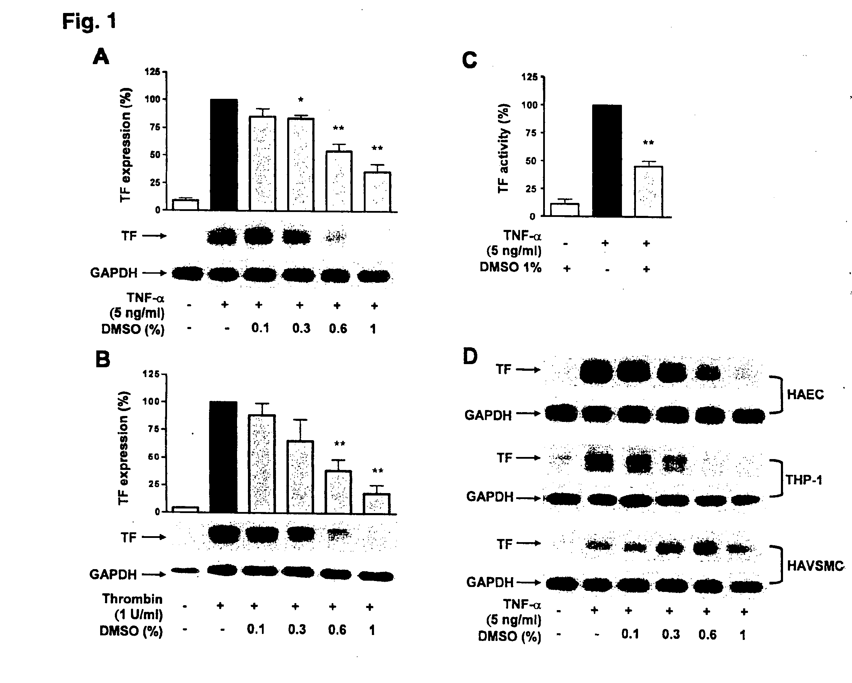

The effect of DMSO on TF protein expression, which was induced by TNF-α, was investigated. Stimulation of HAEC with TNF-α (5 ng/ml) resulted in a 19-fold increase in TF expression, (Fig. 1A); similarly, thrombin (1 U/mL) induced a 24-fold increase (Fig. 1 B). When HAEC were pretreated with increasing concentrations of DMSO (0.1-1%) 1 hour before stimulation, DMSO inhibited TF induction by both TNF-α and thrombin in a concentration-dependent manner (Fig. 1A, B). This effect of DMSO was paralleled by a similar inhibition of TF surface activity (Fig. 1C). Stimulation with TNF-α (5 ng/ml) induced a 10- and 3.5-fold induction of TF expression in THP-1 and HAVSMC, respectively (Fig. 1 D). Treatment with DMSO prevented TF induction in THP-1 cells, but not in HASMC (Fig. 1D).

### Cell culture

Human aortic endothelial cells (HAEC) and human aortic smooth muscle cells (HASMC) (Clonetics, Allschwil, Switzerland) were cultured as described (Steffel, Akhmedov, 2005). THP-1 cells (LGC Prochem) were cultured according to the manufacturer's recommendation. Cells were grown to confluence in 6-cm culture dishes and rendered quiescent for 24 hours before stimulation with 5 ng/mL TNF-α or thrombin 1 U/ml (both from Sigma) for 5 hours. SB203580 (Sigma), PD98059 (Cell Signaling), or SP600125 (Calbiochem) were added 60 minutes before stimulation. To assess cytotoxicity, a colorimetric assay for detection of lactate dehydrogenase (LDH)was used according to the manufacturer's recommendations (Roche); in addition, trypan blue exclusion assay (0.4% solution, Sigma) and morphological examination (Leica, DMIL) was performed.

### Western blot analysis

Protein expression was determined by Western blot analysis as described (Tanner, 2000). Cells were lysed in 50 mmol/L Tris buffer as described (Steffel, Hermann, 2005); 30 µg of sample were loaded, separated by 10% SDS-PAGE, and transferred to a PVDF membrane (Millipore) by semidry transfer. Equal loading was confirmed by Ponceau S staining. Antibodies against human TF, TFPI (both from American Diagnostica) and PAI-1 (SantaCruz Biotec.) were used at 1:2000 dilution. Antibodies against phosphorylated p38 mitogen-activated protein (MAP) kinase (p38), phosphorylated p44/42 MAP kinase (ERK), and phosphorylated JNK (all from Cell Signaling) were used at 1:1000, 1:5000, and 1:1000 dilution, respectively. Antibodies against total p38, total ERK, and total JNK (all from Cell Signaling) were used at 1:2000, 1:10'000, and 1:1000 dilution, respectively. Blots were normalized to GAPDH expression (1:10'000 dilution, Sigma). Representative blots are shown; bars represent at least 3 different experiments.

### TF activity analysis

TF surface activity was analyzed in HAEC with a colorimetric assay (American Diagnostica) according to the manufacturer's recommendations with some modifications as described (Steffel, Akhmedov, 2005). TF/factor Vlla complex converted human factor X to factor Xa, which was measured by its ability to metabolize a chromogenic substrate. Lipidated human TF was used as a positive control to confirm that the results obtained were in the linear range of detection.

TF surface activity was analyzed in carotid artery homogenates employing a colorimetric assay (American Diagnostica) and following the manufacturer's protocol with some modifications that the cells were washed with PBS and were incubated with factor VIIa and factor X at 37°C. Then generation of factor Xa was measured. Right carotid arteries were homogenised in 50 µl of lysis buffer (50 mM Tris-HCl, 100mM NaCl, 0.1 % triton X-100 pH 7.4) and left to stand on ice for 30 minutes. Lipidated human TF was used as a positive control to confirm that the results obtained were in the linear range of detection.

### Example 2: Lack of toxicity of DMSO

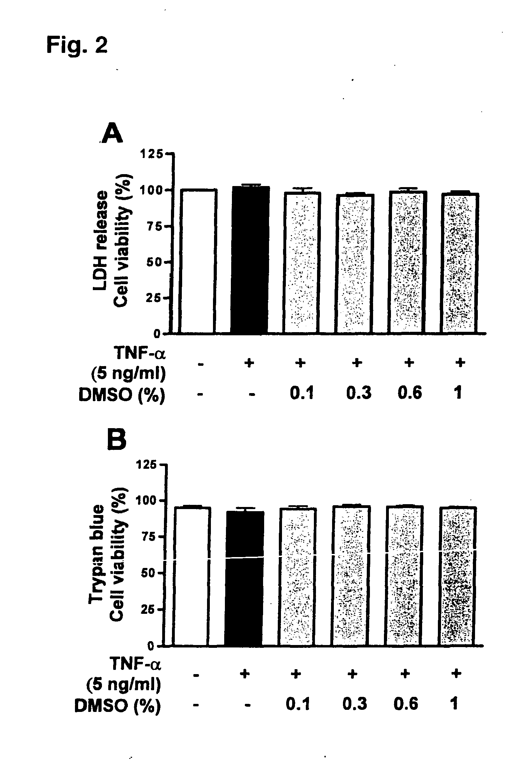

Lack of toxicity of DMSO was investigated. To assess potential toxic effects of DMSO, HAEC were incubated with 1 % DMSO for 6 h, and a morphological examination was performed before and after treatment. No changes in cell morphology were observed under these conditions. In addition, cell death was assessed by LDH release; again, no signs of toxicity could be detected (Fig. 2A). Similarly, Trypan blue staining revealed no increase in the number of stained cells (Fig. 2B). Therefore, treatment of HAEC cells as specified has to be regarded as not toxic.

Cell culture was performed as described in example 1.

### Example 3: Effect of DMSO on TF mRNA expression

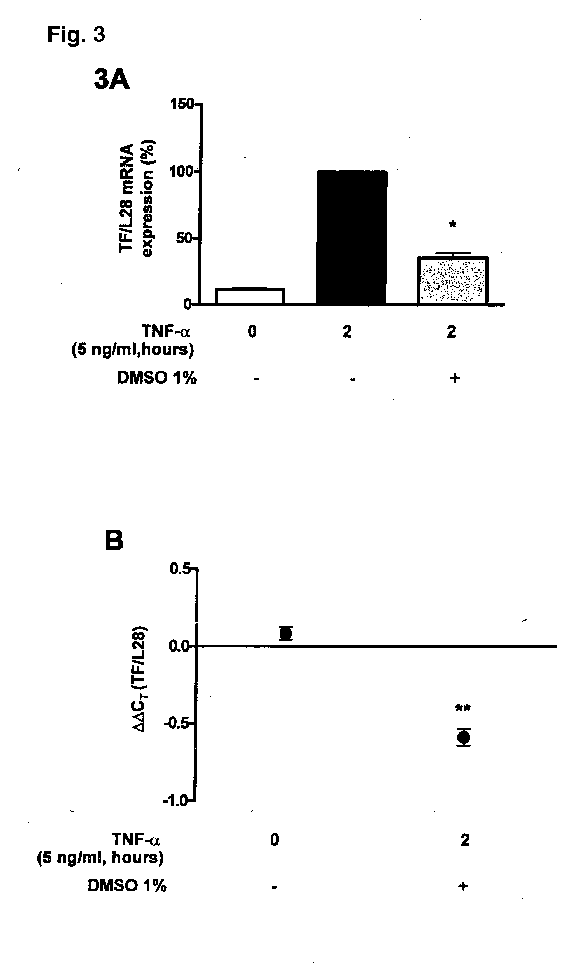

Investigations were performed to which extend DMSO inhibits TF mRNA expression. It is known from literature that peak induction of TF mRNA occurs 2 hours after stimulation with TNF-α (Steffel, Latini, 2005). Real-time PCR analysis was performed and it confirmed a 10 fold induction of TF mRNA after 2 hours of TNF-α stimulation (Fig. 3A). As a result it could be shown that treatment with DMSO (1%) inhibited mRNA synthesis by 65 ± 7% (Fig. 3A, B).

Real-Time PCR analysis was performed as described (Steffel, Latini, 2005). An MX3000P PCR cycler (Stratagene, Amsterdam, The Netherlands) was used according to the manufacturer's instructions. All PCR experiments were performed in triplicates using the SYBR Green JumpStart kit provided by Sigma. Each reaction (25 µl) contained 2 µl cDNA, 1 pmol of each primer, 0.25 µl of internal reference dye and 12.5 µl of JumpStart Taq ReadyMix (containing buffer, dNTPs, stabilizers, SYBR Green, Taq polymerase and JumpStart Taq antibody). The following primers were used. For tissue factor (*F3*): sense primer: 5'-TCCCCAGAGTTCACACCTTACC-3' (bases 508-529 of *F3* cDNA; NCBI no. NM 001993), antisense primer: 5'-TGACCACAAATACCACAGCTCC-3' (bases 892-913 of *F3* cDNA; NCBI no. NM 001993). For human L28: sense primer: 5'-GCATCTGCAATGGATGGT-3', antisense primer: 5'-TGTTCTTGCGGATCATGTGT-3'. The amplification program consisted of 1 cycle at 95°C for 10 min, followed by 40 cycles with a denaturing phase at 95°C for 30 s, an annealing phase of 1 min at 60°C and an elongation phase at 72°C for 1 min. A melting curve analysis was performed after amplification to verify the accuracy of the amplicon. For verification of the correct amplification, PCR products were analyzed on an ethidium bromide stained 1% agarose gel. In each real-time PCR run for F3 and L28 a calibration curve was included, that was generated from serial dilutions (2×10⁷, 2×10⁶, 2×10⁵, 2×10⁴, 2×10³, 2×10³, 2×10², copies/reaction for F3, and 2×10⁷, 2×10⁶, 2×10⁵, 2×10⁴, 2×10³, 2×10³ copies/reaction for L28) of purified amplicons for F3 and L28.

### Example 4: DMSO inhibits TF expression by decreasing MAPK activation

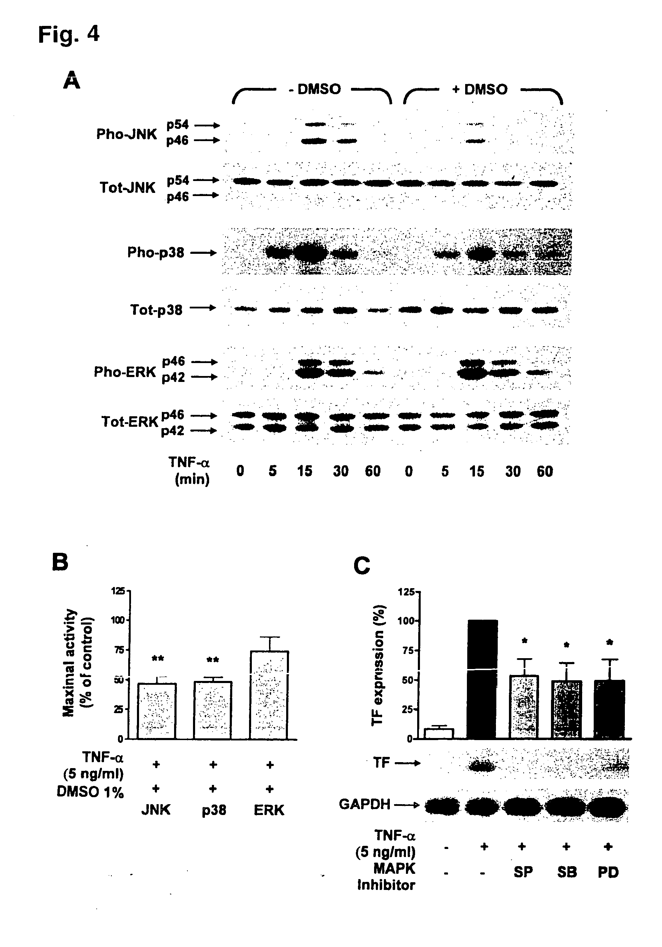

To assess whether DMSO affects MAP kinase activation, HAEC were examined at different time points after stimulation with TNF-α (5 ng/ml). JNK, ERK, and p38 were transiently activated by TNF-α (Fig. 4A).

It could be shown that maximal phosphorylation occurred after 15 minutes and returned to basal levels within 60 minutes (Fig. 4A). DMSO inhibited phosphorylation of JNK and p38 by over 50%, while leaving phosphorylation of ERK unchanged (Fig. 4A). Maximal JNK activation was decreased by 51±6% and that of p38 by 50±3% (Fig. 4B). Maximal activation of ERK was not significantly reduced (Fig. 4B). No change in total expression of p38, ERK, or JNK was observed at any time point with or without DMSO. To verify the involvement of MAP kinases in TF induction by TNF-α under our experimental conditions, the effect of MAP kinase inhibitors on TF expression was examined. SP600125 (10⁻⁶ mol/L), a specific inhibitor of JNK, SB203580 (10⁻⁶ mol/L), a specific inhibitor of p38, and PD98059 (3x10⁻⁷ mol/L), a specific inhibitor of ERK phosphorylation, inhibited TF induction after TNF-α stimulation (Fig. 4C).

Cell culture was performed as described in example 1.

### Example 5: DMSO does not affect TFPI and PAI-1 expression

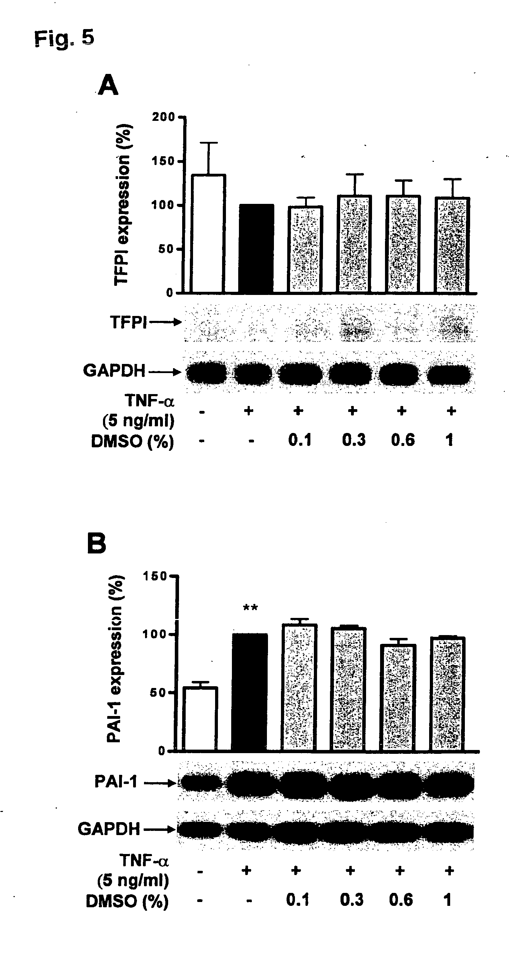

The effect of DMSO on the physiological inhibitor of TF, TFPI, as well as on the anti-fibrinolytic factor, PAI-1, was assessed for a control. It could be shown that treatment of TNF-α stimulated HAEC with DMSO affected neither TFPI nor PAI-1 expression (Fig. 5A, B).

Cell culture was performed as described in example 1 .

### Example 6: DMSO prevents arterial thrombosis in vivo

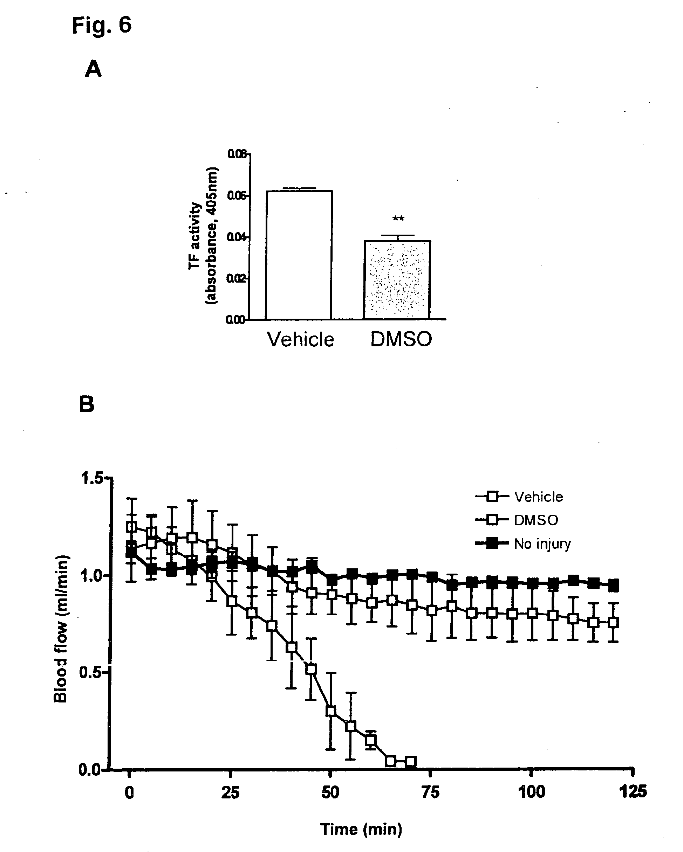

Photochemically induced endothelial injury is an established model of arterial thrombosis and is dependent on TF (Matsuno, 1991). Following onset of injury, vehicle (PBS) treated mice developed thrombotic occlusion within 1 hour (mean occlusion time 63±9.0 minutes, n=5) (Fig. 6A). It can be seen from transient reductions in blood flow that a dynamic state of thrombus formation and lysis occurred during the short time interval prior to complete occlusion. Cessation of blood flow was accompanied by the appearance of a faint white filling inside the lumen of the artery. Thrombotic occlusion was inhibited by treatment with DMSO (3.8 ml/Kg of 40% DMSO in PBS, corresponding to 80 mg/Kg of DMSO). Therefore, it is shown that DSMO prevents arterial thrombosis *in vivo.*

### Carotid artery thrombosis model

C57BU6 mice (6-8 weeks old) weighing an average of 23±2 g were anesthetized with 2 mg intraperitoneal sodium pentobarbital (Butler, Columbus, OH) as previously described (Eitzman, 2000). Rose bengal (Fisher Scientific, Fair Lawn, NJ) was diluted to 10 mg/mL in phosphate-buffered saline and then injected into the tail vein in a volume of 0.12 mL at a concentration of 50 mg/kg using a 27-gauge Precision Glide needle with a 1-mL latex free syringe (Becton Dickinson, Franklin Lakes, NJ). Mice were secured in a supine position, placed under a dissecting microscope (Olympus C-4040 Zoom; spatial resolution 4.1 megapixels), and the right common carotid artery was exposed following a midline cervical incision. A Doppler flow probe (Model 0.5 VB, Transonic Systems, Ithaca, NY) was then applied and connected to a flowmeter (Transonic, Model T106). Exactly 6 minutes after rose bengal injection, a 1.5-mW green light laser (540 nm) (Melles Griot, Carlsbad, CA) was applied to the desired site of injury at a distance of 6 cm for 60 minutes or until thrombosis occurred. From the onset of injury, blood flow in the vessel was monitored for 120 minutes, at which time the experiment was terminated. Occlusion was defined as a flow of ≤0.1 ml/min for at least 1 minute (Eitzman, 2003). Mice were divided into 3 groups: DMSO i.p. (1 hour before surgery, 3.8ml/Kg of 40% DMSO in PBS), PBS i.p., (1 hour before surgery, 3.8ml/Kg of PBS), and negative control (no laser injury).

### Example 7: Proliferation and migration of aortic smooth muscle cells

Proliferation and migration of HASMC were investigated at different concentrations of DMSO.

It could be shown that proliferation and migration of HASMC is decreased with increasing DMSO concentrations.

For proliferation, HASMC were seeded at a density of 10,000 cells per 35-mm dish and cultured for 24 hours, resulting in random proliferation at the beginning of treatment. Dishes were then incubated with different DMSO concentrations and cells were maintained in DMEM (Dulbecco/Vogt Modified Eagle's Minimal Essential Medium) with 20% FCS (Fetal Calf Serum). Medium and appropriate DMSO concentrations were replaced daily and, cell number was determined after 3 days by using a hematocytometer.

For migration, HASMC were seeded at a density of 250,000 cells per 150-mm dish and cultured for 24 hours in DMEM with 20% FCS. Successively, cells were grown in DMEM with 1% FCS for 48 hours. The cells were harvested and resuspended (500'000 cells/ml) for analysis of migration in response to 20% FCS. Migration was assessed in a 2-chamber system (Neuroprobe Inc., Cabin John, Md) as previously described (Tanner, 2004). The number of migrated cells was determined by staining (Diff-Quik; Dade Diagnostics Inc., Aguada, Puerto Rico) and counting the cells at 400x magnification on 4 random microscopic fields per group.

### Literature

- Annex BH, Denning SM, Channon KM, Sketch MH, Jr., Stack RS, Morrissey JH, Peters KG. Differential expression of tissue factor protein in directional atherectomy specimens from patients with stable and unstable coronary syndromes. Circulation. 1995;91:619-22.
- Bardutzky J, Meng X, Bouley J, Duong TQ, Ratan R, Fisher M. Effects of intravenous dimethyl sulfoxide on ischemia evolution in a rat permanent occlusion model. J Cereb Blood Flow Metab. 2005;25:968-77.
- Bourne WM, Shearer DR, Nelson LR. Human corneal endothelial tolerance to glycerol, dimethylsulfoxide, 1,2-propanediol, and 2,3-butanediol. Cryobiology. 1994; 31:1-9.
- Burgess JL, Hamner AP, Robertson WO. Sulfhemoglobinemia after dermal application of DMSO. Vet Hum Toxicol. 1998;40:87-9.
- Day SM, Reeve JL, Myers DD, Fay WP. Murine thrombosis models. Thromb Haemost. 2004; 92:486-94.
- Day SM, Reeve JL, Pedersen B, Farris DM, Myers DD, lm M, Wakefield TW, Mackman N, Fay WP. Macrovascular thrombosis is driven by tissue factor derived primarily from the blood vessel wall. Blood. 2005;105:192-8.
- Dujovny M, Rozario R, Kossovsky N, Diaz FG, Segal R. Antiplatelet effect of dimethyl sulfoxide, barbiturates, and methyl prednisolone. Ann N YAcad Sci. 1983; 411:234-44.
- Egorin MJ, Rosen DM, Sridhara R, Sensenbrenner L, Cottler-Fox M. Plasma concentrations and pharmacokinetics of dimethylsulfoxide and its metabolites in patients undergoing peripheral-blood stem-cell transplants. J Clin Oncol. 1998;16:610-5.
- Eitzman DT, Bodary PF, Shen Y, Khairallah CG, Wild SR, Abe A, Shaffer-Hartman J, Shayman JA. Fabry disease in mice is associated with age-dependent susceptibility to vascular thrombosis. J Am Soc Nephrol. 2003;14:298-302.
- Eitzman DT, Westrick RJ, Xu Z, Tyson J, Ginsburg D. Plasminogen activator inhibitor-1 deficiency protects against atherosclerosis progression in the mouse carotid artery. Blood. 2000;96:4212-5.
- Eto M, Kozai T, Cosentino F, Joch H, Luscher TF. Statin prevents tissue factor expression in human endothelial cells: role of Rho/Rho-kinase and Akt pathways. Circulation. 2002;105:1756-9.
- Farkas E, Institoris A, Domoki F, Mihaly A, Luiten PG, Bari F. Diazoxide and dimethyl sulphoxide prevent cerebral hypoperfusion-related learning dysfunction and brain damage after carotid artery occlusion. Brain Res. 2004;1008:252-60.
- Heidari Y, Shah AM, Gove C. NOX-2S is a new member of the NOX family of NADPH oxidases. Gene. 2004;335:133-40.
- lwasaki T, Hamano T, Aizawa K, Kobayashi K, Kakishita E. A case of pulmonary amyloidosis associated with multiple myeloma successfully treated with dimethyl sulfoxide. Acta Haematol. 1994;91:91-4.
- Jo SK, Hu X, Yuen PS, Aslamkhan AG, Pritchard JB, Dear JW, Star RA. Delayed DMSO administration protects the kidney from mercuric chloride-induced injury. J Am Soc Nephrol. 2004;15:2648-54.
- Karaca M, Bilgin UY, Akar M, de la Torre JC. Dimethly sulphoxide lowers ICP after closed head trauma. Eur J Clin Pharmacol. 1991;40:113-4.
- Karaca M, Kilic E, Yazici B, Demir S, de la Torre JC. Ischemic stroke in elderly patients treated with a free radical scavenger-glycolytic intermediate solution: a preliminary pilot trial. Neurol Res. 2002;24:73-80.
- Kopp CW, Holzenbein T, Steiner S, Marculescu R, Bergmeister H, Seidinger D, Mosberger I, Kaun C, Cejna M, Horvat R, Wojta J, Maurer G, Binder BR, Breuss JM, Ecker RC, de Martin R, Minar E. Inhibition of restenosis by tissue factor pathway inhibitor: in vivo and in vitro evidence for suppressed monocyte chemoattraction and reduced gelatinolytic activity. Blood. 2004;103:1653-61.
- Lind RC, Gandolfi AJ. Late dimethyl sulfoxide administration provides a protective action against chemically induced injury in both the liver and the kidney. Toxicol Appl Pharmacol. 1997;142:201-7.
- Mackman N. Role of tissue factor in hemostasis, thrombosis, and vascular development. Arterioscler Thromb Vasc Biol. 2004;24:1015-22.
- Maier W, Altwegg LA, Corti R, Gay S, Hersberger M, Maly FE, Sutsch G, Roffi M, Neidhart M, Eberli FR, Tanner FC, Gobbi S, von Eckardstein A, Luscher TF. Inflammatory markers at the site of ruptured plaque in acute myocardial infarction: locally increased interleukin-6 and serum amyloid A but decreased C-reactive protein. Circulation. 2005;111:1355-61.
- Mann KG, van't Veer C, Cawthern K, Butenas S. The role of the tissue factor pathway in initiation of coagulation. Blood Coagul Fibrinolysis. 1998;9 Suppl 1:S3-7.
- Matsuno H, Uematsu T, Nagashima S, Nakashima M. Photochemically induced thrombosis model in rat femoral artery and evaluation of effects of heparin and tissue-type plasminogen activator with use of this model. J Pharmacol Methods. 1991;25:303-17.
- McCammon KA; Lentzner AN, Moriarty RP, Schellhammer PF. lntravesical dimethyl sulfoxide for primary amyloidosis of the bladder. Urology. 1998;52:1136-8.
- Mechtcheriakova D, Schabbauer G, Lucerna M, Clauss M, De Martin R, Binder BR, Hofer E. Specificity, diversity, and convergence in VEGF and TNF-alpha signaling events leading to tissue factor up-regulation via EGR-1 in endothelial cells. Faseb J. 2001;15:230-242.
- Moons AH, Levi M, Peters RJ. Tissue factor and coronary artery disease. Cardiovasc Res. 2002;53:313-25.
- Morassi P, Massa F, Mesesnel E, Magris D, D'Agnolo B. [Treatment of amyloidosis with dimethyl sulfoxide (DMSO)]. Minerva Med. 1989;80:65-70.
- Nemerson Y. Tissue factor and hemostasis. Blood. 1988;71:1-8.
- Okada H, Woodcock-Mitchell J, Mitchell J, Sakamoto T, Marutsuka K, Sobel BE, Fujii S. Induction of plasminogen activator inhibitor type 1 and type 1 collagen expression in rat cardiac microvascular endothelial cells by interleukin-1 and its dependence on oxygen-centered free radicals. Circulation. 1998;97:2175-82.
- Pedersen B, Holscher T, Sato Y, Pawlinski R, Mackman N. A balance between tissue factor and tissue factor pathway inhibitor is required for embryonic development and hemostasis in adult mice. Blood. 2005;105:2777-82.
- Salim AS. Role of oxygen-derived free radical scavengers in the management of recurrent attacks of ulcerative colitis: a new approach. J Lab Clin Med. 1992;119:710-7.
- Salim AS. Role of free radical scavengers in the management of refractory duodenal ulceration. A new approach. J Surg Res. 1994;56:45-52.
- Steffel J, Akhmedov A, Greutert H, Luscher TF, Tanner FC. Histamine induces tissue factor expression: implications for acute coronary syndromes. Circulation. 2005; 112:341-9.
- Steffel J, Hermann M, Greutert H, Gay S, Luscher TF, Ruschitzka F, Tanner FC. Celecoxib decreases endothelial tissue factor expression through inhibition of c-Jun terminal NH2 kinase phosphorylation. Circulation. 2005;111:1685-9.
- Steffel J, Latini RA, Akhmedov A, Zimmermann D, Zimmerling P, Luscher TF, Tanner FC. Rapamycin, but not FK-506, increases endothelial tissue factor expression: implications for drug-eluting stent design. Circulation. 2005;112:2002-11.
- Steffel J, Luscher TF, Tanner FC. Tissue factor in cardiovascular diseases: molecular mechanisms and clinical implications. Circulation. 2006;113:722-31.
- Tanner FC, Largiader T, Greutert H, Yang Z, Luscher TF. Nitric oxide synthase gene transfer inhibits biological features of bypass graft disease in the human saphenous vein. J Thorac Cardiovasc Surg. 2004;127:20-6.
- Tanner FC, Meier P, Greutert H, Champion C, Nabel EG, Luscher TF. Nitric oxide modulates expression of cell cycle regulatory proteins: a cytostatic strategy for inhibition of human vascular smooth muscle cell proliferation. Circulation. 2000;101:1982-9.
- Wong CK, Lin CS. Remarkable response of lipoid proteinosis to oral dimethyl sulphoxide. Br J Dermatol. 1988;119:541-4.

## Claims

1. Clinical device for being implanted, **characterized in that** if comprises means for eluting an active ingredient, said active ingredient essentially comprising DMSO.

2. Clinical device according to claim 1, wherein at least 80% of the active ingredient is DMSO.

3. Clinical device according to claims 1 or 2, wherein at least 95% of the active ingredient is DMSO.

4. Clinical device according to claims 1 to 3, **characterized in that** it contains means for eluting the active ingredient into the surrounding tissue.

5. Clinical device according to claims 1 to 4, **characterized in that** it contains from about 10 ng to about 10 mg of active ingredient.

6. Clinical device according to claims 1 to 5, wherein the active ingredient is eluted within a predetermined period of time of from about 1 to about 28 days.

7. Clinical device according to claims 1 to 6, **characterized in that** the eluted amount of DMSO is effective to significantly suppress expression and/or activity of tissue factor protein in the surrounding tissue and/or effective to significantly suppress proliferation and/or migration of human vascular smooth muscle cells.

8. Clinical device according to claims 1 to 7, **characterized in that** the amount of DMSO eluted into the surrounding tissue is from 1 nmol/mm²h to about 1 µmol/mm²h.

9. Clinical device according to claims 1 to 8, **characterized in that** the amount of DMSO eluted is such that the concentration of DMSO in the surrounding tissue is from about 1 nmol/L to about 1 mmol/L.

10. Clinical device according to claims 1 to 9, **characterized in that** the means for eluting an amount of DMSO into the surrounding tissue is a polymer matrix.

11. Clinical device according to any one of the preceding claims which is a stent.

12. Use of DMSO for the manufacture of a medicament for the treatment of vascular, cerebrovascular or cardiovascular disorders, acute coronary syndromes, stroke or peripheral artery disease.

13. Use of DMSO for the manufacture of a medical device for the treatment of vascular, cerebrovascular or cardiovascular disorders, acute coronary syndromes, stroke or peripheral artery disease.

14. Method of treating vascular, cerebrovascular or cardiovascular disorders, stroke, acute coronary syndromes or peripheral artery disease by administration of a drug-eluting stent able to release a drug comprising a significant amount of DMSO and/or by administering a medicament comprising a significant amount of DMSO to a patient in need thereof.

15. Method of reducing restenosis, in-stent restenosis, stent thrombosis or in-stent thrombosis comprising providing a drug-eluting stent able to release a drug comprising a significant amount of DMSO and/or administering a medicament comprising a significant amount of DMSO to a patient in need thereof.

16. Method of treating acute myocardial infarction, stroke or peripheral artery disease comprising administering a medicament comprising a significant amount of DMSO to a subject receiving a stent in connection with percutaneous transluminal angioplasty.
